(19)

**Europäisches Patentamt**

**European Patent Office**

**Office européen des brevets**

(11) **EP 4 194 471 A1**

(12) **EUROPEAN PATENT APPLICATION**

(43) Date of publication:
**14.06.2023 Bulletin 2023/24**

(21) Application number: **21383131.6**

(22) Date of filing: **10.12.2021**

(51) International Patent Classification (IPC):
**C07K 16/30** (2006.01)    **A61P 35/00** (2006.01)
**A61K 39/00** (2006.01)

(52) Cooperative Patent Classification (CPC):
**C07K 16/3084; C07K 16/3053;** A61K 2039/505;
C07K 2317/24

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Y-Mabs Therapeutics, Inc.**
**New York NY 10169 (US)**
• **Hospital Sant Joan de Deu**
**08950 Esplugues de Llobregat Barcelona (ES)**
• **Memorial Sloan-Kettering Cancer Center**
**New York, NY 10065 (US)**
• **Sloan-Kettering Institute for Cancer Research**
**New York, NY 10065 (US)**
• **Memorial Hospital for Cancer and Allied Diseases**
**New York, NY 10065 (US)**

(72) Inventors:
• **MORA GRAUPERA, Jaume**
**08950 ESPLUGUES DE LLOBREGAT (ES)**
• **CHEUNG, Nai-Kong**
**NEW YORK, 10022 (US)**
• **MODAK, Shakeel**
**NEW YORK, 10028 (US)**
• **RAJAH, Vignesh**
**2950 VEDBÆK (DK)**
• **MØLLER SAN-PEDRO, Claus J.**
**3250 GILLELEJE (DK)**

(74) Representative: **Valua**
**Valua ApS**
**Borgmester Jensens Allé 25C, 1. sal**
**2100 Copenhagen (DK)**

Remarks:
The complete document including Reference Table(s) and the Sequence Listing(s) can be downloaded from the EPO website

(54) **ANTI-GD2 ADMINISTRATION REGIMEN**

(57)    The present invention relates to the administration of GD2 binding antibodies associated with side-effects. In particular, the present invention concerns a method for reducing side-effects associated with the administration of a GD2 binding antibody.

**EP 4 194 471 A1**

**Description**

The present invention relates to the administration of GD2 binding antibodies associated with side-effects. In particular, the present invention concerns a method for reducing side-effects associated with the administration of a GD2 binding antibody.

Technical Background

Ganglioside GD2-binding antibodies are found to be promising immunotherapeutic agents against GD2-expressing tumors such as neuroblastomas. GD2 expression in normal human tissue is restricted to skin melanocytes, neurons, and peripheral nerve fibers, and the high expression of GD2 in neuroblastomas makes GD2 a tumor-associated antigen suitable for immunotherapy. Using GD2-binding antibodies will mediate tumor cell death through antibody-dependent cellular cytotoxicity (ADCC) and complement dependent cytotoxicity (CDC).

In the publication: "Pathogenesis of the neurotoxicity caused by anti-GD2 antibody therapy", Journal of the Neurological Sciences, vol. 149, 1 August 1997, pages 127-130, Yuki et al. reported side effects associated with administration of anti-GD2 MAb (14G2a).

Despite a promising antitumor effect caused by immunotherapy using GD2 antibodies, a substantial toxicity burden is recognized. Patients receiving GD2 antibody treatment experience severe adverse effect including intense pain, fever, allergic reactions, fluctuating blood pressure, and neurotoxicity (Blom, Thomas et al. "Treatment-Related Toxicities During Anti-GD2 Immunotherapy in High-Risk Neuroblastoma Patients." Frontiers in oncology vol. 10 601076. 17 Feb. 2021, doi:10.3389/fonc.2020.601076). Blom et al. performed a non-systematic literature review on toxicity associated with anti-GD2 antibody immunotherapy in high-risk neuroblastoma patients. The immunotherapy protocol consisted of administration of dinutuximab with the dosage 17.5 mg/m$^2$ per day as a 10 hours intravenous constant rate administration for 4 consecutive days, or dinutuximab beta with the dosage 20 mg/m$^2$ per day as an 8 hours constant rate administration for 5 consecutive days. The most common adverse effects observed was pain, fever, coughing, edema, and liver enzyme abnormalities..

In the publication: "Humanized 3F8 Anti-GD2 Monoclonal Antibody Dosing with Granulocyte-Macrophage Colony-Stimulating Factor in Patients with Resistant Neuroblastoma A Phase 1 Clinical Trial, JAMA Oncology vol. 4, 20 September 2018, pages 1729-1735, Kushner et al. conducted a phase 1 clinical trial using a 3+3 dose escalation design for the administration of hu3F8. Each cycle comprised of administration of GM-CSF at 250 $\mu$g/m$^2$/d on days -4 to 0, followed by a stepping-up of GM-CSF to 500 $\mu$g/m$^2$/d on days 1 to 5. Hu3F8 was administered with a constant rate intravenously for 30 minutes on days 1, 3, and 5. The patients were premedicated with opiates and antihistamines to limit adverse effects.

In the publication: "A Phase II Trial of Hu14.18K322A in Combination with Induction Chemotherapy in Children with Newly Diagnosed High-Risk Neuroblastoma", Clinical Cancer Research, vol. 25, November 2019, Furman et al. investigates the combination of GD2 antibody hu14.18K322A with induction chemotherapy and the responses and outcomes in children with newly diagnosed neuroblastoma. hu14.18K322A was administered in 4 daily doses to each course of induction chemotherapy on days 2-5. The dosage of hu14.18K322A was fixed at 40 mg/m$^2$ per dose. Each dose of hu14.18K322A was planned to be administered over 4 hours, however due to patient tolerance, the hu14.18K322A administration was extended to 8 or 16 hours for some patients. The patients were also receiving continuous infusions of narcotics such as morphine, hydromorphone, or fentanyl at standard dosage 30 minutes before hu14.18K322A administration. Each course was followed by daily administration of 250 mg/m$^2$ GM-CSF subcutaneously. The adverse effects recorded include pain, hypotension, cough, and hypoxia.

In the publication: "A Pilot Trial of Hu14.18-IL2 (EMD 273063) in Subjects with Completely Resectable Recurrent Stage III or Stage IV Melanoma", Cancer Immunology Immunotherapy, vol. 67, October 2018, pages 1647-1658, Albertini et al. conducted a phase I clinical trial testing adjuvant hu14.18-IL2 in melanoma patients. The patients received hu14.18-IL2 on days 1, 2, and 3. The daily dose administered was 6 mg/m$^2$/day, and each dose was administered intravenously for 4 hours at a continuous rate. The adverse effects of hu14.18-IL2 treatment recorded was hypotension, pain, and elevated bilirubin and creatinine.

In the publication: "Phase I trial of murine monoclonal antibody 14G2a administered by prolonged intravenous infusion in patients with neuroectodermal tumors", Journal of Clinical Oncology, vol. 12, 1 January 1994, pages 184-193, Murray et al. investigated the toxicity and maximum-tolerated dose of 14G2a in patients with neuroectodermal tumors such as melanoma, neuroblastoma, or osteosarcoma. The patients received 50, 100 or 200 mg/m$^2$/day of 14G2a intravenously for 24 hours for 5 days. The toxicity and adverse effect from prolonged 14G2a administration consisted of hypotension, pain, hyponatremia, fever, and rash. Murray et al. concluded: "Mab 14G2a has modest antitumor activity at the expense of significant toxicity."

The adverse effects such as pain, fever, rash, and capillary leak syndrome are most likely due to increased production of inflammatory peptides. The severe adverse effects limit the dose of GD2 antibody that can be administered.

The optimal administration schedule and timing must yet be determined (Navid, Fariba et al. "Anti-GD2 antibody therapy for GD2-expressing tumors." Current cancer drug targets vol. 10,2 (2010): 200-9).

Summary of the invention

The administration of GD2 antibodies gives rise to severe adverse effects, in particular pain, hypotension and/or hypoxia. Specifically, intravenous administration of anti-ganglioside GD2 monoclonal antibodies in humans causes pain irrespective of mouse derived 3F8 and 14.G2a, chimeric ch14.18 (dinutuximab or dinutuximab beta), or humanized (naxitamab or hu14.18K322A). This pain remains the major drawback of anti-GD2 therapy and a major dose limiting toxicity in the development of all anti-GD2 antibodies. Pain is usually severe, diffuse, and mostly visceral and its mechanism still uncertain. Partly because of the pain side effects and the remedies given to patients, anti-GD2 antibody infusions commonly cause hypotension, tachycardia, urticaria, pyrexia, bronchospasm, cough, vomiting, and nausea.
Known adverse events are summarized in Table 1.

Table 1: Adverse events as a result of anti-GD2 immunotherapy

| Adverse event N=48 | Any grade %(n) | Grade 3 or 4 %(n) |
| --- | --- | --- |
| Hypotension | 98% (47) | 63% (30) |
| Pain | 96% (46) | 65% (31) |
| Urticaria | 83% (40) | 29% (14) |
| Pyrexia | 79% (38) | 2% (1) |
| Bronchospasm | 67% (32) | 21% (10) |
| Tachycardia | 63% (30) | 2% (1) |
| Cough | 58% (28) | 0 |
| Vomiting | 52% (25) | 2% (1) |
| Nausea | 50% (24) | 2% (1) |
| Abdominal pain | 46% (22) | 15% (7) |
| Neutropenia | 23% (11) | 15% (7) |
| Depressed level of consciousness | 21% (10) | 10% (5) |
| Hypoxia | 10% (5) | 10% (5) |

The adverse effects may be remedied by pre-administration of opiates and steroids to reduce pain and inflammation, and antihistamines to reduce the risks of anaphylaxis. Alternatively, the administration of GD2 antibodies have been extended using very long infusion time in order to reduce (but not eliminating) adverse effects, which is not ideal for patients consequentially enduring very long infusion times. Reducing complement activation using K322A mutation also failed to eliminate pain since patients continued to require substantial narcotics for pain control ("Phase I Trial of a Novel Anti-GD22 Monoclonal Antibody, Hu14.18K322A, Designed to Decrease Toxicity in Children With Refractory or Recurrent Neuroblastoma", J. Clin. Oncol. 32: 1445-1452, 2014).
There is therefore a need for an improved administration regime for GD2 antibodies that reduces the adverse reactions and do not require too long infusion periods.
Anti-GD2 antibodies cause major side effects including pain, hypotension and hypoxia, which are significant management challenges especially in young children. These side effects are electrophysiologic when sensory neurons are activated through antibody binding. Based on clinical signs and symptoms as well as electrophysiologic studies (described below), these side effects are likely mediated through the Autonomous Nervous System. It is also known that most of the small unmyelinated C-fibers in the human body belong to the Autonomous Nervous System, whereas the A$\alpha$-, A$\beta$- or A$\delta$- fibers are mainly believed to be part of the Somatic Nervous System.
The inventors have conducted experiments using confocal microscopy of tissue samples from healthy subjects, where the GD2 antigen has been fluorescently labelled. The GD2 antigen is mainly found in the perineurium and endoneurium of peripheral nerves including the most prominent autonomic nervous system nerve, the vagus.
Anti-GD2 experiments in the rat model have shown that unmyelinated C-fiber nerves are those mainly activated by anti-GD2 antibodies. In the human body, C-fiber umyelinated nerves belong mostly to the autonomic nervous system and most prominently in the vagus nerve.

Without wishing to be bound by theory, given the sites of pain (visceral), the timing of pain onset (within 30 minutes of antibody infusion), the systemic effects (tachycardia, hypotension), uncommon organ damage (including nerves), the ineffectiveness of opioids or $\alpha2\delta$ calcium channel blockers (gabapentin or neurontin), and the absence of pathologic evidence of immune neuritis, a possible explanation of the anti-GD2 antibody side effects is activation of the autonomic nervous system through the vagus nerve. When anti-GD2 antibody infusion was slowed down, the pain side effect may decrease but often persists. When antibody infusion was given over 30 min to an hour, the side effects are severe. But soon after the antibody infusion, the side effects will resolve, despite the continual presence of circulating anti-GD2 antibodies in the blood. This is consistent with the phenomenon of tachyphylaxis, whereby electric firing from neurons becomes increasingly blunted with activation until they stop firing completely. To recreate tachyphylaxis, anti-GD2 antibodies are given in a dose step-up fashion, starting with a small dose just enough to fire the most sensitive but small number of neurons, followed by a bigger dose to fire the next layer but still small number of neurons, and so on until most of the neurons have been blunted before the full dose of antibody is given. The benefits provided by the methods described in the present invention is not limited to the particular example of anti-GD2 antibody, but would apply likewise to any antibody, protein or chemical having similar binding properties and neuronal activation properties, e.g. 3F8, hu3F8 (naxitamab), 14G2a, ch14.18 (dinutuximab, dinutuximab-beta), hu14.18-K322A, hu14.18-IL2, hu3F8-BsAb (nivatrotamab), and hu3F8-SADA.

The treating doctor may determine a suitable dose depending on the actual circumstances. Typical, the total daily dose is determined based on the weight or the body surface area of the patient.

The administered total daily dose of GD2 antibody can be calculated using the formulas:

$$\text{Administered dose (mg/day)} = \text{dose (mg/kg/day)} * \text{body weight (kg)},$$

or

$$\text{Administered dose (mg/day)} = \text{dose (mg/m}^2\text{/day)} * \text{body surface area (m}^2\text{)}.$$

Naxitamab is a GD2 antibody that was recently approved by the FDA under the name of DANYELZA . According to the package label as approved by the FDA, it is recommended that the administration of Naxitamab in young children at the age of 24 months with different body weights, may occur with the following doses and injection volumes illustrated in table 2, given that the recommended dose is 3 mg/kg/day and the dose in each vial is 4 mg/mL.

Tabel 2: Examples of administered dose and volume of Naxitamab in children (24 months)

| Weight (kg) | Dose (mg/day) | Injected volume (mL) |
|---|---|---|
| 12 | 36 | 9 |
| 13 | 39 | 9.75 |
| 15 | 45 | 11.25 |
| 20 | 60 | 15 |

The body surface area (BSA) can be estimated based on the height (cm) and weight (kg) using the Mosteller equation:

$$\text{BSA (m2)} = \sqrt{\frac{\text{height (cm)} * \text{weight (kg)}}{3600}}$$

Examples of BSAs of young children at the age of 24 months with different body weights and heights are illustrated in table 3:

Table 3: Examples of BSA of children (24 months)

| Weight (kg) | Height (cm) | BSA (m$^2$) |
|---|---|---|
| 12 | 81 | 0,520 |

(continued)

| Weight (kg) | Height (cm) | BSA (m$^2$) |
|---|---|---|
| 13 | 85 | 0,554 |
| 15 | 90 | 0,612 |
| 20 | 95 | 0,726 |

The present invention provides a GD2 antibody administration regimen consisting of increasing administration rates, preferably step-wise, resulting in reduced adverse effect, particularly infusion reactions such as pain and hypotension. In addition, the requirement of premedication to manage adverse effects is reduced as well as the need of nurses and doctors to safely manage the patients during administration.

Aspects of the present intervention are provided in the items as well as the independent claims.

Detailed Disclosure

Embodiments of the present intervention are provided in the items as well as the claims.

According to an embodiment, the present invention may relate to Self-Assembly and DisAssembly (SADA) technology that was originally described in the International patent application with publication number WO 2018204873A1, incorporated herein by reference. The technology is based on SADA-domains, small polypeptides that has the property of self-assembly and disassembly depending inter alia on concentration. In some embodiments, a SADA polypeptide is or comprises a tetramerization domain of p53, p63, p73, hnRNPC, SNAP-23, Stefin B, KCNQ4, CBFA2T1 or any of the other examples of such polypeptides provided in said international patent application, without limitation.

According to an embodiment, the present invention may relate to a SADA-complex. According to the present specification, a SADA-complex is intended to mean a polypeptide comprising a SADA domain and at least one additional domain.

According to an embodiment, the present invention may relate to a SADA-complex comprising a GD2 binding site.

Definitions

**Antibody or antibody fragment**

An antibody fragment is a portion of an antibody such as F(ab')2, F(ab)2, Fab', Fab, Fv, sFv and the like. Regardless of structure, an antibody fragment binds with the same antigen that is recognized by the intact antibody. For example, an 3F8 monoclonal antibody fragment binds with an epitope recognized by 3F8. The term "antibody fragment" also includes any synthetic or genetically engineered protein that acts like an antibody by binding to a specific antigen to form a complex. For example, antibody fragments include isolated fragments consisting of the variable regions, such as the "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region.

**Antibody**

The term "antibody" is art-recognized terminology and is intended to include molecules or active fragments of molecules that bind to known antigens. Examples of active fragments of molecules that bind to known antigens include Fab and F(ab')2 fragments. These active fragments can be derived from an antibody of the present invention by a number of techniques. For example, purified monoclonal antibodies can be cleaved with an enzyme, such as pepsin, and subjected to HPLC (high-performance liquid chromatography) gel filtration. The appropriate fraction containing Fab fragments can then be collected and concentrated by membrane filtration and the like. The term "antibody" also includes bispecific and chimeric antibodies and other available formats.

**Antibody fragment**

An antibody fragment is a portion of an antibody such as F(ab')2, F(ab)2, Fab', Fab, Fv, sFv and the like. Regardless of structure, an antibody fragment binds with the same antigen that is recognized by the intact antibody. For example, an 3F8 monoclonal antibody fragment binds with an epitope recognized by 3F8. The term "antibody fragment" also includes any synthetic or genetically engineered protein that acts like an antibody by binding to a specific antigen to form a complex. For example, antibody fragments include isolated fragments consisting of the variable regions, such as the "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region.

**Bispecific antibody**

A bispecific antibody is an antibody that can bind simultaneously to two targets which are of different structure. Bispecific antibodies (bsAb) and bispecific antibody fragments (bsFab) have at least one arm that specifically binds to an antigen, for example, GD2 and at least one other arm that specifically binds to another antigen, for example a targetable conjugate that bears a therapeutic or diagnostic agent. A variety of bispecific fusion proteins can be produced using molecular engineering. In one form, the bispecific fusion protein is divalent, consisting of, for example, a scFv with a single binding site for one antigen and a Fab fragment with a single binding site for a second antigen. In another form, the bispecific fusion protein is tetravalent, consisting of, for example, an IgG with two binding sites for one antigen and two identical scFv for a second antigen.

**CDR**

Complementarity Determining Regions (CDR) are part of the variable regions of antibodies and are of key importance for the binding specificity of an antibody. A typically antibody consisting of two heavy chains and two light chains has 6 CDR sequences, three in the light chain and three in the heavy chain.

**Chimeric antibody**

A chimeric antibody is a recombinant protein that contains the variable domains including the complementarity-determining regions (CDRs) of an antibody derived from one species, for example a rodent antibody, while the constant domains of the antibody molecule is derived from those of a human antibody. The constant domains of the chimeric antibody may also be derived from that of other species, such as a cat or dog.

**Humanized antibody**

A humanized antibody is a recombinant protein in which the CDRs from an antibody from one species; e.g., a rodent antibody, is transferred from the heavy and light variable chains of the rodent antibody into human heavy and light variable domains. The constant domain of the antibody molecule is derived from those of a human antibody.
A human antibody may be an antibody obtained from transgenic mice that have been "engineered" to produce specific human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas.

**Subject**

By "subject" or "individual" or "animal" or "patient" or "mammal," is meant any subject, particularly a mammalian subject, for whom diagnosis, prognosis, or therapy is desired. Mammalian subjects include humans and other primates, domestic animals, farm animals, and zoo, sports, or pet animals such as dogs, cats, guinea pigs, rabbits, rats, mice, horses, cattle, cows, and the like.

**Treatment**

As used herein, the terms "treatment", "treat", "treated" or "treating" refer to prophylaxis and/or therapy, particularly wherein the object is to prevent or slow down (lessen) an undesired physiological change or disorder, such as the progression of multiple sclerosis. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

**Pharmaceutical composition**

As used herein the term "Pharmaceutical composition " is intended to mean a composition for administration as a drug or medicine to a patient in need thereof. Pharmaceutical compositions are prepared from pharmaceutical grade ingredients e.g. as described in European Pharmacopoeia 10th Edition, using methods and technologies known in the pharmaceutical or apothecary area.

**SADA**

The self-assembly and disassembly (SADA) technology was originally disclosed in WO 2018204873A1, and make use of SADA domains having the property of assembly and disassembly depending on concentration. Complexes comprising a SADA domain typically exists in at least two distinct forms, a tetrameric form at high concentration and a monomeric form at low concentration.

Figures

Figure 1 shows the result of confocal microscopy of a tissue sample of the vagus nerve. It is concluded that the GD2 antigen is mainly found connected to the unmyelinated C-fibers (grey area).

Figure 2 shows the result of the electrophysiological analysis of the sudomotor response during GD2 antibody (Naxitamab) infusion of day 1. The sudomotor response decreases with time as a result of continuous infusion with GD2 antibody.

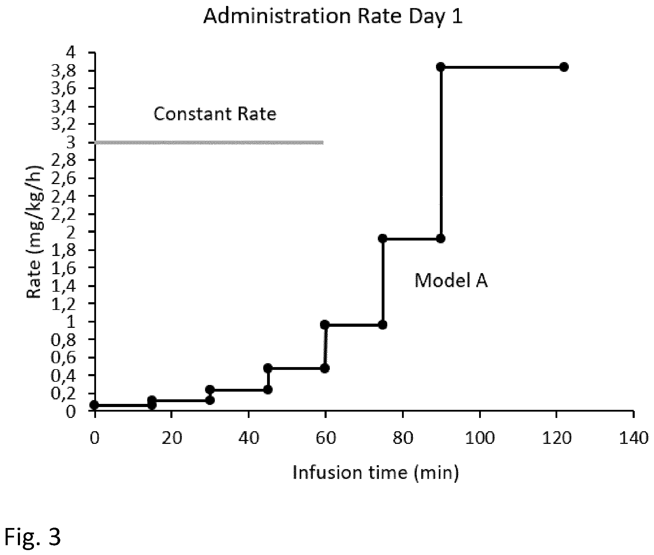

Figure 3 shows the GD2 antibody administration rate and the total infusion time of model A compared to a constant administration rate of day 1.

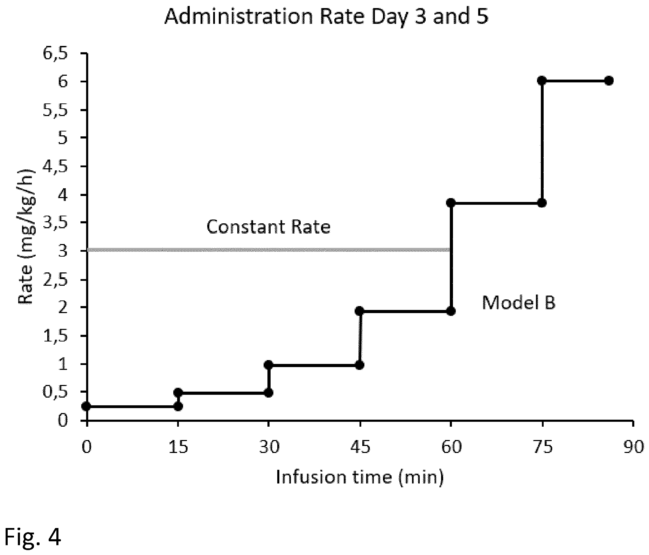

Figure 4 shows the GD2 antibody administration rate and the total infusion time of model B compared to a constant administration rate for each of day 3 and 5.

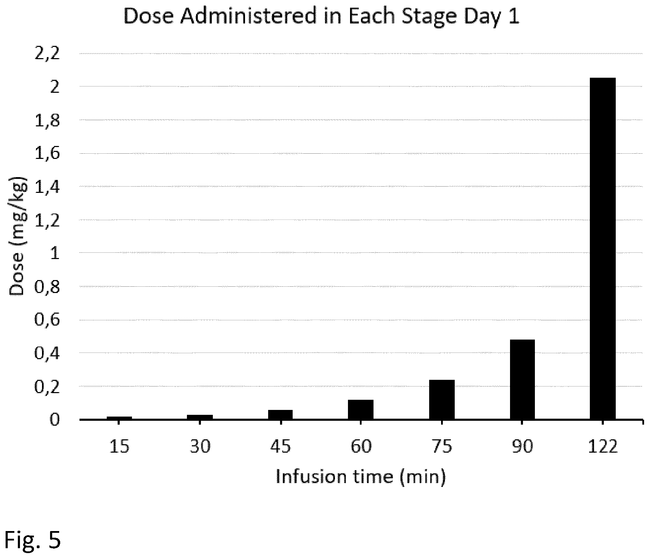

Figure 5 shows the administered GD2 antibody dose for each stage and the total infusion time from the beginning of infusion for model A of day 1.

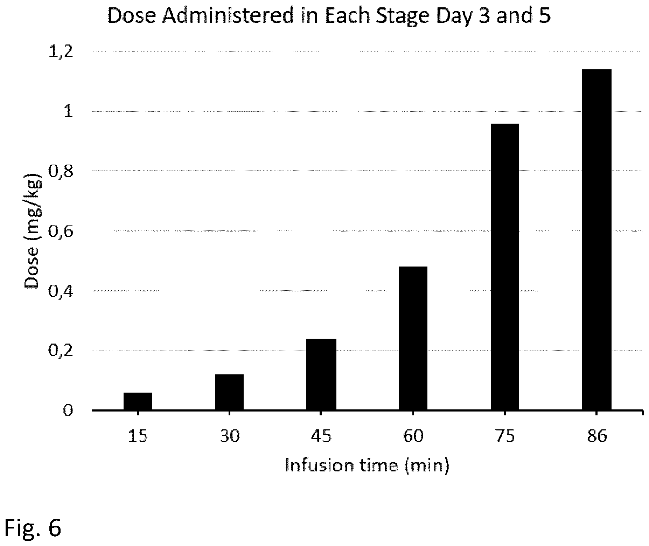

Figure 6 shows the administered GD2 antibody dose for each stage and the total infusion time from the beginning of infusion for model B for each of day 3 and 5.

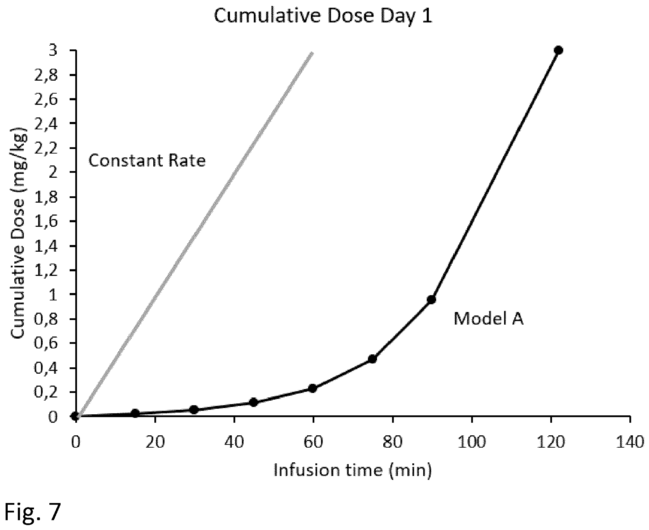

Figure 7 shows the administered cumulative dose and the total Infusion time of day 1 for model A compared to a constant administration rate. For model A, each data point represents the cumulative dose of GD2 binding antibody at a specific time point. The total time of administration of the total dosage is no more than 4 hours.

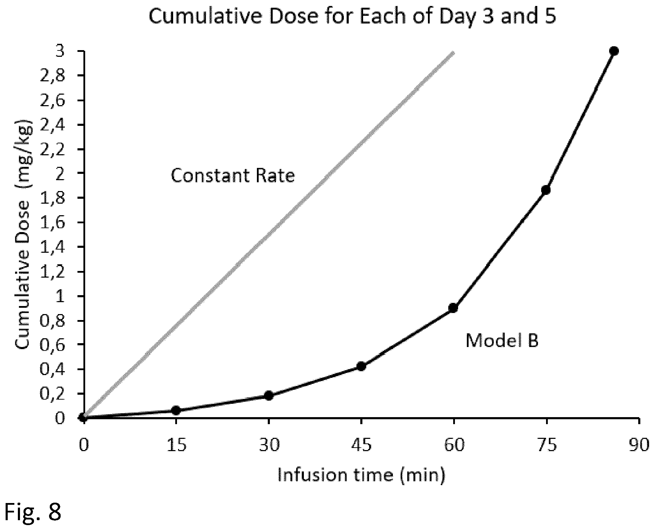

Figure 8 shows the administered cumulative dose and the total Infusion time for each of day 3 and 5 for model B compared to a constant administration rate. For model B, each data point represents the cumulative dose of GD2 binding antibody at a specific time point. The total time of administration of the total dosage is no more than 4 hours.

All cited references are incorporated by reference.

The accompanying Figures and Examples are provided to explain rather than limit the present invention. It will be clear to the person skilled in the art that aspects, embodiments, claims and any items of the present invention may be combined.

Unless otherwise mentioned, all percentages are in weight/weight. Unless otherwise mentioned, all measurements are conducted under standard conditions (ambient temperature and pressure). Unless otherwise mentioned, test conditions are according to European Pharmacopoeia 8.0.

Examples

Materials and Methods

GD2-antibody: Naxitamab, commercially available as DANYELZA (Y-mAbs Therapeutics Inc, NY;USA)

**Example 1 - Confocal microscopy**

Methods

We performed anatomical (confocal microscopy) analyses in primary human tissues and neuroblastoma patients to localize the GD2 antigens in these tissues.
Anti-GD2 monoclonal antibodies were stained with a green fluorescent dye and applied to the tissues, whereafter the samples were inspected using confocal microscopy.

Results

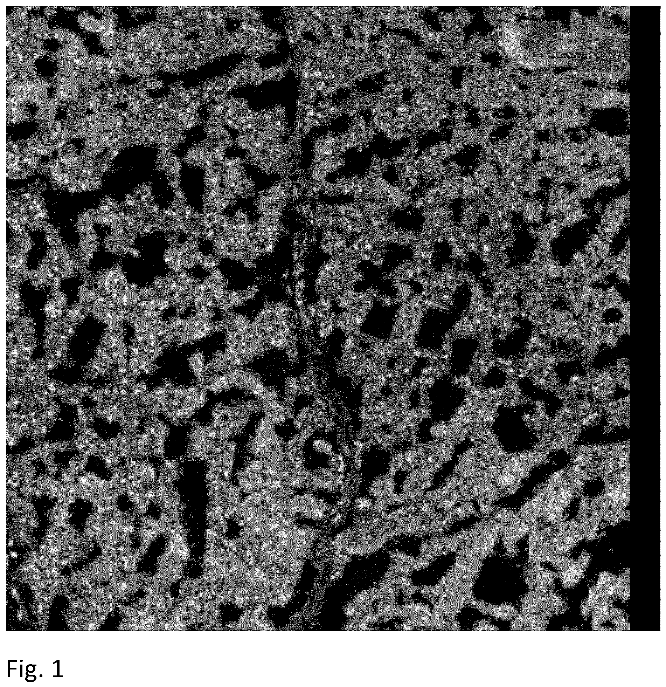

Confocal microscopy of peripheral nerves from the autonomous nerve system of human autopsies of neonates placed GD2 expression in the Schwann cells of the endoneurium but not in the neuronal axons (see figure 1). The expression levels were estimated to be approximately 4 times lower than GD2 expression of neuroblastoma tumor cells.

**Example 2** - **Electrophysiological analysis**

Electrophysiological analysis of the autonomous nerve system responses were measured during GD2 antibody infusion in neuroblastoma patients. The patient was treated with GD2 antibody infusion in a number of cycles, where each cycle consisted of infusions on day 1 (Monday), 3 (Wednesday) and 5 (Friday).
For this example the electrophysiological analysis was performed during infusions that took place on day 1 of the cycle.

Methods:

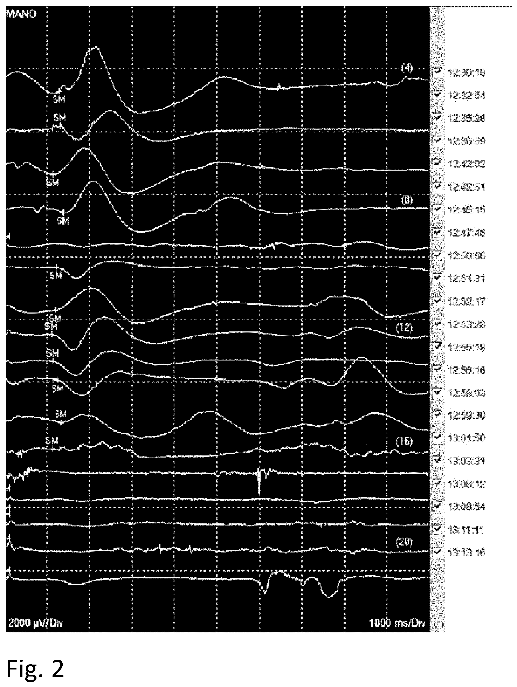

Electrophysiological (Sympathetic Skin Response (SSR)) analyses in primary human tissues and neuroblastoma patients were performed to demonstrate the activation of the autonomous nervous system as the underlying cause of most adverse events incurred during GD2 antibody administration
SSR was used to measure the sudomotor and cardiovascular components of autonomous nerve system activation, and action potentials analyzed for latency and amplitude following sympathetic stimulation.
SSR was performed to evaluate the unmyelinated axon function of the autonomous nerve system.
For measurements of electrodermal activity following sympathetic stimulation with a surface electromyography, electrodes were placed on the patient's palm or sol and a reference electrode.
A baseline response was recorded and infusion of GD2 was started and the measured responses recorded during the complete infusion (see figure 2).

Results:

In all cases response showed shorter latency and lower amplitudes immediately after infusion start.
Progressive decrease of responses until complete disappearance some time after treatment initiation.
Responses never recovered after 45 minutes of treatment.

**Example 3** - **Electrophysiological analysis**

The experiment disclosed in Example 3 was repeated on infusions performed on day 5 of a cycle.
The results showed a lower amplitude to begin and faster loss of response.

**Example 4 - The stepping-up protocol.**

A new protocol for infusion of GD2 antibody was developed and tested in 20 patients.
According to the protocol, patients were treated in cycles containing infusion of GD2-antibody on day 1 (Monday), 3 (Wednesday) and 5 (Friday).

For Day 1 following infusion scheme was used:

Table 4: GD2 antibody administration rate of day 1

| Step# | | Infusion time | Rate | Dose administered in each step | Volume administered in each step |
|---|---|---|---|---|---|
| 1 | 1 mL/h | 15 minutes | 0.06 mg/kg/h | 0.015 mg/kg | 0.25 mL |
| 2 | 2 mL/h | 15 minutes | 0.12 mg/kg/h | 0.03 mg/kg | 0.50 mL |
| 3 | 4 mL/h | 15 minutes | 0.24 mg/kg/h | 0.06 mg/kg | 1.00 mL |
| 4 | 8 mL/h | 15 minutes | 0.48 mg/kg/h | 0.12 mg/kg | 2.00 mL |
| 5 | 16 mL/h | 15 minutes | 0.96 mg/kg/h | 0.24 mg/kg | 4.00 mL |
| 6 | 32 mL/h | 15 minutes | 1.92 mg/kg/h | 0.48 mg/kg | 8.00 mL |
| 7 | 64 mL/h | 32 minutes | 3.84 mg/kg/h | 2.05 mg/kg | 34.24 mL |
| | Total | 122 minutes (2.04 h) | | 3.00 mg/kg | 50.0 mL |
| Note that the "Volume administered in each step" is based on the assumption that the total dose to be given is <80mg. | | | | | |

For day 3 and 5 the following infusion scheme was used:

Table 5: GD2 antibody administration rate for each of day 3 and 5

| Step# | | Infusion time | Rate | Dose administered in each step | Volume administered in each step |
|---|---|---|---|---|---|
| 1 | 4 mL/h | 15 minutes | 0.24 mg/kg/h | 0.06 mg/kg | 1.00 mL |
| 2 | 8 mL/h | 15 minutes | 0.48 mg/kg/h | 0.12 mg/kg | 2.00 mL |
| 3 | 16 mL/h | 15 minutes | 0.96 mg/kg/h | 0.24 mg/kg | 4.00 mL |
| 4 | 32 mL/h | 15 minutes | 1.92 mg/kg/h | 0.48 mg/kg | 8.00 mL |
| 5 | 64 mL/h | 15 minutes | 3.84 mg/kg/h | 0.96 mg/kg | 16.00 mL |
| 6 | 100 mL/h | 11 minutes | 6.00 mg/kg/h | 1.14 mg/kg | 19.00 mL |
| | Total | 86 minutes (1.44 h) | | 3.00 mg/kg | 50.0 mL |
| Note that the "Volume administered in each step" is based on the assumption that the total dose to be given is <80mg. | | | | | |

This stepping-up protocol was tested in 20 patients for a total of 53 cycles, 159 infusions. 3 patients (15%) presented Grade 3 or 4 adverse events (hypertension, apnea, and pain). This protocol permitted a decrease in the level of monitoring required, with no MD at the bedside necessary.

This has to be compared with infusion with constant rate usually applied and were applied in clinical trials Study 201 where Pain in grade 3 or 4 were experienced by 72% of the patients.

Sequences

Naxitamab sequences:
SEQ ID NO: 1 Naxitamab Heavy Chain

QVQLVESGPGVVQPGRSLRISCAVSGFSVTNYGVHWVRQPPGKGLEWLGVIWAGGITNYNSAFMSRLT

ISKDNSKNTVYLQMNSLRAEDTAMYYCASRGGHYGYALDYWGQGTLVTVSSASTKGPSVFPLAPSSKSTS

GGTAALGCLVKDYFPEPVTVSWNSGALTSGVHTFPAVLQSSGLYSLSSVVTVPSSSLGTQTYICNVNHKPS

NTKVDKRVEPKSCDKTHTCPPCPAPELLGGPSVFLFPPKPKDTLMISRTPEVTCVVVDVSHEDPEVKFNW

YVDGVEVHNAKTKPREEQYNSTYRVVSVLTVLHQDWLNGKEYKCKVSNKALPAPIEKTISKAKGQPREPQ

VYTLPPSRDELTKNQVSLTCLVKGFYPSDIAVEWESNGQPENNYKTTPPVLDSDGSFFLYSKLTVDKSRWQ

QGNVFSCSVMHEALHNHYTQKSLSLSPGK

SEQ ID NO: 2 Naxitamab Light Chain

EIVMTQTPATLSVSAGERVTITCKASQSVSNDVTWYQQKPGQAPRLLIYSASNRYSGVPARFSGSGYGTE

FTFTISSVQSEDFAVYFCQQDYSSFGQGTKLEIKRTVAAPSVFIFPPSDEQLKSGTASVVCLLNNFYPREAKV

QWKVDNALQSGNSQESVTEQDSKDSTYSLSSTLTLSKADYEKHKVYACEVTHQGLSSPVTKSFNRGEC

SEQ ID NO: 3 Naxitamab VH

QVQLVESGPGVVQPGRSLRISCAVSGFSVTNYGVHWVRQPPGKGLEWLGVIWAGGITNYNSAFMSRLT

ISKDNSKNTVYLQMNSLRAEDTAMYYCASRGGHYGYALDYWGQGTLVTVSS

SEQ ID NO: 4 Naxitamab VL

EIVMTQTPATLSVSAGERVTITCKASQSVSNDVTWYQQKPGQAPRLLIYSASNRYSGVPARFSGSGYGTE

FTFTISSVQSEDFAVYFCQQDYSSFGQGTKLEIK

CDR Sequence for Naxitamab:
SEQ ID NO: 5 Naxitamab VH CDR1
NYGVH
SEQ ID NO: 6 Naxitamab VH CDR2
VIWAGGITNYNSAFMS
SEQ ID NO: 7 Naxitamab VH CDR3
GGHYGYALDY
SEQ ID NO: 8 Naxitamab VL CDR1
KASQSVSNDVT
SEQ ID NO: 9 Naxitamab VL CDR2
SASNRYS
SEQ ID NO: 10 Naxitamab VL CDR3
QQDYSS
SEQ ID NO: 11 GD2-SADA

EIVMTQTPATLSVSAGERVTITCKASQSVSNDVTWYQQKPGQAPRLLIYSASNRYSGVPARFSGSGYGTE
FTFTISSVQSEDFAVYFCQQDYSSFGCGTKLEIKRGGGGSGGGGSGGGGSGGGGSGGGGSGGGGSQVQ
LVESGPGVVQPGRSLRISCAVSGFSVTNYGVHWVRQPPGKCLEWLGVIWAGGITNYNSAFMSRLTISKD
NSKNTVYLQMNSLRAEDTAMYYCASRGGHYGYALDYWGQGTLVTVSSGGGGSGGGGSGGGGSGGGG
GSHVQLVESGGGLVQPGGSLRLSCAASGFSLTDYGVHWVRQAPGKGLEWLGVIWSGGGTAYNTALISR
FTISRDNSKNTLYLQMNSLRAEDTAVYYCARRGSYPYNYFDAWGCGTLVTVSSGGGGSGGGGSGGGGS
GGGGSGGGGSGGGGSQAVVTQEPSLTVSPGGTVTLTCGSSTGAVTASNYANWVQQKPGQCPRGLIGG
HNNRPPGVPARFSGSLLGGKAALTLLGAQPEDEAEYYCALWYSDHWVIGGGTKLTVLGTPLGDTTHTSG
KPLDGEYFTLQIRGRERFEMFRELNEALELKDAQAGKEPGGSGGA

SEQ ID NO: 12 Dinutuximab Heavy Chain

EVQLLQSGPELEKPGASVMISCKASGSSFTGYNMNWVRQNIGKSLEWIGAIDPYYGGTSYNQKFKGRAT
LTVDKSSSTAYMHLKSLTSEDSAVYYCVSGMEYWGQGTSVTVSSASTKGPSVFPLAPSSKSTSGGTAALG
CLVKDYFPEPVTVSWNSGALTSGVHTFPAVLQSSGLYSLSSVVTVPSSSLGTQTYICNVNHKPSNTKVDKR
VEPKSCDKTHTCPPCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQPREPQVYTLPPSR
EEMTKNQVSLTCLVKGFYPSDIAVEWESNGQPENNYKTTPPVLDSDGSFFLYSKLTVDKSRWQQGNVFS
CSVMHEALHNHYTQKSLSLSPGK

SEQ ID NO: 13 Dinutuximab Light Chain

EIVMTQSPATLSVSPGERATLSCRSSQSLVHRNGNTYLHWYLQKPGQSPKLLIHKVSNRFSGVPDRFSGS
GSGTDFTLKISRVEAEDLGVYFCSQSTHVPPLTFGAGTKLELKRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSPVTK
SFNRGEC

Items

1. A method of treatment and/or diagnosis comprising the administration to a subject of a dosage of a GD2 binding antibody, said method comprising at least the steps of:

    i. Administering said antibody with a first administration rate in a first period of time, followed by
    ii. Administering said antibody with a second administration rate in a second period of time, wherein said second administration rate is higher than said first administration rate.

2. The method according to item 1, wherein said method is for treating a human subject for a condition treatable with GD2 binding antibodies.
3. The method according to any of the preceding items wherein said method is for diagnosis of a condition associated with elevated expression of GD2.
4. The method according to any of the preceding items wherein said administration is conducted by infusion.
5. The method according to any of the preceding items, wherein said first administration rate is sufficiently high to allow at least some systemic nerves to become stimulated, but sufficiently low to substantially avoid unwanted side-effects.
6. The method according to any of the preceding items, wherein said first administration rate is sufficiently high to allow sympathetic nerves to become stimulated, but sufficiently low to substantially avoid unwanted side-effects.

7. The method according to any of the preceding items, wherein said first administration rate is sufficiently high to allow at least some receptors binding to said antibody to get saturated, but sufficiently low to substantially avoid side-effects.

8. The method according to any of the preceding items, wherein said antibody interacts with nerves, such as sympathetic nerves.

9. The method according to any of the preceding items, wherein the GD2 binding antibody is a complete antibody consisting of two heavy chains and two light chains, a bispecific antibody, a SADA construct, an antibody fragment selected among F(ab')2, F(ab)2, Fab', Fab, Fv, scFv and the like, or a chimeric and/or recombinant construct comprising a GD2 binding site.

10. The method according to any of the preceding items, wherein the GD2 binding antibody is a immunocytokine, such as hu14.18-IL2.

11. The method according to any of the preceding items, wherein the GD2 binding antibody is humanized.

12. The method according to any of the preceding items, wherein the GD2 binding antibody comprises the CDR sequences of SEQ ID NO: 5, 6, 7, 8, 9 and 10.

13. The method according to any of the preceding items, wherein the GD2 binding antibody comprises the VH sequence of SEQ ID NO: 3 and the VL sequence of SEQ ID NO: 4.

14. The method according to any of the preceding items, wherein the GD2 binding antibody comprises the heavy chain sequence of SEQ ID NO: 1 and the light chain sequence of SEQ ID NO: 2.

15. The method according to any of the preceding items, wherein the GD2 binding antibody is a SADA construct, and preferably comprises the sequence of SEQ ID NO: 11.

16. The method according to any of the preceding items, wherein the GD2 binding antibody is dinutuximab; comprises the sequence of SEQ ID NO: 12 and the sequence of SEQ ID NO: 13; and/or comprises the CDR sequences of dinutuximab.

17. The method according to any of the preceding items, wherein the method is for the treatment or amelioration of cancer.

18. The method of item 17, wherein the cancer is selected among: neuroblastoma, melanoma, osteosarcoma, sarcoma, brain tumor, small cell lung cancer, retinoblastoma, thyroid cancer, breast cancer or carcinoma.

19. The method of item 17, wherein said cancer is selected among osteosarcoma, liposarcoma, fibrosarcoma, malignant fibrous histiocytoma, leiomyosarcoma, spindle cell sarcoma, brain tumor, small cell lung cancer, retinoblastoma, HTLV-1 infected T cell leukemia, triple negative breast cancer and other GD2 positive tumors.

20. The method according to any of the preceding items, wherein the number of adverse reactions in grade G3 or G4 is reduced compared to the situation wherein the complete administered dosage of the GD2 binding antibody is administered with a constant administration rate in a period corresponding to said first period or time plus said second period of time.

21. The method of item 20, wherein the number of adverse reactions in grade G3 or G4 is reduced by at least 40%, preferably at least 50%, preferably at least 60%, preferably at least 70%, preferably at least 80%, preferably at least 90% or wherein the number of adverse reactions in grade G3 and G4 is practically absent.

22. The method according to any of the preceding items, wherein the administration rate is gradually, continuously or stepwise increased during the total time of administration.

23. The method according to any of the preceding items, wherein the total time of administration of said dosage is no more than 4 hours, and the amount of antibody administered during the first 15 minutes of administration is no more than 5%, preferably no more than 4%, more preferred no more than 3%, preferably no more than 2% of the total amount of said antibody.

24. The method according to any of the preceding items, wherein the total time of administration of said dosage is no more than 4 hours, and the amount of antibody administered during the first 30 minutes of administration is no more than 10%, preferably no more than 8%, more preferred no more than 6% of the total amount of said antibody.

25. The method according to any of the preceding items, wherein the total time of administration of said dosage is no more than 4 hours, and the amount of antibody administered during the first 45 minutes of administration is no more than 30%, preferably no more than 20%, more preferred no more than 15% of the total amount of said antibody.

26. The method according to any of the preceding items, wherein no more than 10% of the total amount of said antibody is administered during the first 15%, more preferred the first 20%, preferably the first 25%, more preferred the first 30%, preferably the first 33% of the total time of administration of said dosage.

27. The method according to any of the preceding items, wherein no more than 33% of the total amount of said antibody is administered during the first 50%, more preferred the first 55%, preferably the first 60%, more preferred the first 65%, preferably the first 70% of the total time of administration of said dosage.

28. The method according to any of the preceding items, wherein at least 50% of the total amount of said antibody is administered during the last 40%, more preferred the last 35%, preferably the last 30%, more preferred the last 25%, preferably the last 20% of the total time of administration of said dosage.

29. The method according to any of the preceding items, wherein at least 66% of the total amount of said antibody is administered during the last 50%, more preferred the last 45%, preferably the last 40%, more preferred the last 35%, preferably the last 33% of the total time of administration of said dosage.

30. The method according to any of the preceding items, where the treatment is performed in two or more cycles, wherein each cycle comprises administration of GD2 antibody on two or more days, and each cycle spans seven days.

31. The method according to any of the preceding items, where the treatment is performed in two or more cycles, where each cycle comprises administration of GD2 antibody on two or more days, where two administration days are separated by one day without antibody administration, and two cycles separated by at least two days without antibody administration.

32. The method of item 30 or 31, wherein each cycle comprises three days of antibody administration.

33. The method according to any of the preceding items, wherein the administration of said dosage is preceded by at least two days without administration of said antibody.

34. The method according to item 33, wherein no more than 20% of the total dosage is administered during the first 75 minutes of administration.

35. The method according to item 33 or 34, wherein at least 80% of the total dosage is administered during the last 60 minutes of administration.

36. The method according to any of the preceding items, wherein the antibody is administered with infusion, and the infusion rate is adjusted within the following stages:

| Stage | Infusion Time (minutes) | Rate (mg/kg/h) |
|---|---|---|
| 1 | 15 | 0.06 |
| 2 | 15 | 0.12 |
| 3 | 15 | 0.24 |
| 4 | 15 | 0.48 |
| 5 | 15 | 0.96 |
| 6 | 15 | 1.92 |
| 7 | 32 | 3.84 |

37. The method according to any of the items 33 - 36, wherein the antibody is administered with infusion, and the infusion rate is adjusted to $\pm 90\%$, more preferred $\pm 80\%$, preferably $\pm 70\%$, more preferred $\pm 60\%$, preferably $\pm 50\%$, more preferred $\pm 40\%$, preferably $\pm 30\%$, more preferred $\pm 20\%$, preferably $\pm 10\%$ of the rate of item 36, and the infusion is continued until the administration of the dosage has been completed.

38. The method according to any of the items 33 - 37, wherein the antibody is administered with infusion, and the infusion rate is adjusted to the rate of item 36 or 37, and the infusion time is adjusted to $\pm 90\%$, more preferred $\pm 80\%$, preferably $\pm 70\%$, more preferred $\pm 60\%$, preferably $\pm 50\%$, more preferred $\pm 40\%$, preferably $\pm 30\%$, more preferred $\pm 20\%$, preferably $\pm 10\%$ of the infusion time of item 36, and the infusion is continued until the administration of the dosage has been completed.

39. The method according to any of the preceding items, wherein the antibody is administered with infusion, and the dose is administered in the following stages:

| Stage | Infusion Time (minutes) | Dose administered in stage (mg/kg) |
|---|---|---|
| 1 | 15 | 0.015 |
| 2 | 15 | 0.03 |
| 3 | 15 | 0.06 |
| 4 | 15 | 0.12 |
| 5 | 15 | 0.24 |
| 6 | 15 | 0.48 |
| 7 | 32 | 2.05 |

40. The method according to any of the items 33 - 39, wherein the antibody is administered with infusion, and the dose is adjusted to $\pm 90\%$, more preferred $\pm 80\%$, preferably $\pm 70\%$, more preferred $\pm 60\%$, preferably $\pm 50\%$, more preferred $\pm 40\%$, preferably $\pm 30\%$, more preferred $\pm 20\%$, preferably $\pm 10\%$ of the dose of item 39, and the infusion is continued until the administration of the dosage has been completed.

41. The method according to any of the items 33 - 40, wherein the antibody is administered with infusion, and the dose is adjusted to the dose of item 39 or 40, and the infusion time is adjusted to $\pm 90\%$, more preferred $\pm 80\%$, preferably $\pm 70\%$, more preferred $\pm 60\%$, preferably $\pm 50\%$, more preferred $\pm 40\%$, preferably $\pm 30\%$, more preferred $\pm 20\%$, preferably $\pm 10\%$ of the infusion time of item 39, and the infusion is continued until the administration of the dosage has been completed.

42. The method according to any of the preceding items, wherein the administration of said dosage is conducted no more than 2 days after the prior administration of said antibody.

43. The method according to item 42, wherein no more than 20% of the total dosage is administered during the first 45 minutes of administration.

44. The method according to item 42 or 43, wherein at least 70% of the total dosage is administered during the last 30 minutes of administration.

45. The method according to any of the preceding items, wherein the antibody is administered with infusion, and the infusion rate is adjusted within the following stages:

| Stage | Infusion Time (minutes) | Rate (mg/kg/h) |
| --- | --- | --- |
| 1 | 15 | 0.24 |
| 2 | 15 | 0.48 |
| 3 | 15 | 0.96 |
| 4 | 15 | 1.92 |
| 5 | 15 | 3.84 |
| 6 | 11 | 6.00 |

46. The method according to any of the items 42 - 45, wherein the antibody is administered with infusion, and the infusion rate is adjusted to ±90%, more preferred ±80%, preferably ±70%, more preferred ±60%, preferably ±50%, more preferred ±40%, preferably ±30%, more preferred ±20%, preferably ±10% of the rate of item 45, and the infusion is continued until the administration of the dosage has been completed.

47. The method according to any of the items 42 - 46, wherein the antibody is administered with infusion, and the infusion rate is adjusted to the rate of item 45 or 46, and the infusion time is adjusted to ±90%, more preferred ±80%, preferably ±70%, more preferred ±60%, preferably ±50%, more preferred ±40%, preferably ±30%, more preferred ±20%, preferably ±10% of the infusion time of item 45, and the infusion is continued until the administration of the dosage has been completed.

48. The method according to any of the preceding items, wherein the antibody is administered with infusion, and the dose is administered in the following stages:

| Stage | Infusion Time (minutes) | Dose administered in stage (mg/kg) |
| --- | --- | --- |
| 1 | 15 | 0.06 |
| 2 | 15 | 0.12 |
| 3 | 15 | 0.24 |
| 4 | 15 | 0.48 |
| 5 | 15 | 0.96 |
| 6 | 11 | 1.14 |

49. The method according to any of the items 42 - 48, wherein the antibody is administered with infusion, and the dose is adjusted to ±90%, more preferred ±80%, preferably ±70%, more preferred ±60%, preferably ±50%, more preferred ±40%, preferably ±30%, more preferred ±20%, preferably ±10% of the dose of item 48, and the infusion is continued until the administration of the dosage has been completed.

50. The method according to any of the items 42 - 49, wherein the antibody is administered with infusion, and the dose is adjusted to the dose of item 48 or 49, and the infusion time is adjusted to ±90%, more preferred ±80%, preferably ±70%, more preferred ±60%, preferably ±50%, more preferred ±40%, preferably ±30%, more preferred ±20%, preferably ±10% of the infusion time of item 48, and the infusion is continued until the administration of the dosage has been completed.

51. The method according to any of the preceding items, wherein the antibody is administered with infusion, and the infusion rate is adjusted according to item 36 or 45.

52. The method according to any of the preceding items, wherein the antibody is administered with infusion, and the dose is administered according to item 39 or 48.

53. The method according to any of the preceding items, wherein the total time of administration is not more than 7 hours, preferably not more than 6 hours, more preferred not more than 5 hours, preferably not more than 4 hours, more preferred not more than 3.5 hours, preferably not more than 3 hours, more preferred not more than 2.5 hours.

54. The method according to any of the preceding items, wherein the total time of administration is more than 60 minutes, preferably more than 70 minutes, more preferred more than 75 minutes, preferably more than 80 minutes, more preferred more than 85 minutes.

55. The method according to any of the preceding items, wherein the concentration of the antibody administered in step i. is lower than the concentration of the antibody administered in step ii.

56. The method according to any of the preceding items, wherein said first period of time lasts until the degree of nerve stimulation decreases.

57. The method according to any of the preceding items, wherein said first period of time is at least 30 minutes, more preferred 45 minutes, preferably 60 minutes, more preferred 75 minutes, preferably at least 120 minutes.

58. The method according to any of the preceding items, wherein the dosage administered during step i. is 0.5-50%, 1-20%, preferably 2-15%, more preferred 3-12%, preferably 4-10%, more preferred 5-8%, preferably 6% w/w of the total dosage.

59. The method according to any of the preceding items, wherein the first administration rate is selected so that dosage administered during step i. is 0.5-50%, 1-20%, preferably 2-15%, more preferred 3-12%, preferably 4-10%, more preferred 5-8%, preferably 6% w/w of the total dosage.

60. The method according to any of the preceding items, wherein the first administration rate is below 1 mg/kg/h.

61. The method according to any of the preceding items, wherein the first administration rate is selected in the range of 0.05-1 mg/kg/h, preferably 0.1-0.5 mg/kg/h, preferably around 0.3 mg/kg/h.

62. The method according to any of the preceding items, wherein the first period is longer than 60 minutes, preferably in the range of 60-120 minutes, preferably in the range of 60 - 90 minutes and most preferred around 75 minutes.

63. The method according to any of the preceding items, wherein said second administration rate is sustained until the administration of said dosage has been completed.

64. The method according to any of the preceding items, wherein said second period of time is no more than 15 minutes, more preferred 30 minutes, preferably 45 minutes, more preferred 60 minutes, preferably at 90 minutes.

65. The method according to any of the preceding items, wherein the second administration rate is selected so that dosage administered during step ii. is at least 80% of the total administered dosage, preferably at least 90% or the total dosage, and most preferred around 94% of the total dosage.

66. The method according to any of the preceding items, wherein the second administration rate is above 1 mg/kg/h, preferably in the range of 1-5 mg/kg/h, and most preferred around 4 mg/kg/h.

67. The method according to any of the preceding items, wherein the second period is 15 minutes or longer, preferably in the range of 15-30 minutes.

68. The method according to any of the preceding items, wherein the total amount of GD2 binding antibody is administered in step i and ii.

69. The method according to any of the preceding items, wherein the total administered dosage of GD2 binding antibody is in the range of 50-150 mg.

70. The method according to any of the preceding items, wherein the GD2 binding antibody is naxitamab, the first period is 75 minutes and the first administration rate is selected so 6% of the total administered dosage of GD2 binding antibody is administered in step i, and the rest of the total administered dosage is administered by infusion during step ii.

71. The method according to any of the preceding items, further comprising a break between step i and step ii.

72. The method according to any of the preceding items, wherein the subject is a human adult, a juvenile or a child.

73. The method according of any of the preceding items, wherein the subject of said administration is a human or animal subject.

74. The method according of any of the preceding items, wherein the subject of said administration is a human adult, a juvenile or a child.

75. An antibody for use in a method of treatment according to any of the preceding items.

76. A GD2 binding antibody or fragment thereof for use in a method of treatment according to any of the preceding items.

77. A pharmaceutical composition comprising an antibody for use in a method of treatment according to any of the preceding items.

78. The pharmaceutical composition according to item 77, comprising an excipient, binder or diluent.

79. Use of an antibody for the manufacture of a pharmaceutical composition according to item 77 or 78.

80. A GD2 binding antibody for use in a method of treatment comprising the steps of:

>    i. Administering said GD2 binding antibody with a first administration rate in a first period of time, followed by
>    ii. Administering said GD2 binding antibody with a second administration rate in a second period of time, wherein said second administration rate is higher than said first administration rate.

81. The GD2 binding antibody of item 80, wherein said method is for the treatment or amelioration of a condition in a subject, wherein said condition is affected by the administration of said antibody.

82. The GD2 binding antibody of item 80 or 81, wherein said first administration rate is sufficiently high to allow systemic nerves to become stimulated, but sufficiently low to substantially avoid unwanted side-effects.

83. The GD2 binding antibody according to any of the items 80 - 82, wherein said first administration speed is sufficiently high to allow receptors to get saturated, but sufficiently low to substantially avoid side-effects.

84. The GD2 binding antibody according to any of the items 80 - 83, wherein the number of adverse reactions in grade G3 or G4 is reduced compared to the situation wherein the complete administered dosage of the GD2 binding antibody is administered with a constant administration rate in a period corresponding to said first period or time plus said second period of time.

85. The GD2 binding antibody of item 84, wherein the number of adverse reactions in grade G3 or G4 is reduced with at least 40%, preferably at least 50%, preferably at least 60%, preferably at least 70%, preferably at least 80%, preferably at least 90% or wherein the number of adverse reactions in grade G3 and G4 is practically absent.

86. The GD2 binding antibody according to any of the items 80 - 85, wherein the method comprises treatment of cancer.

87. The GD2 binding antibody of item 86, wherein the cancer is selected among:
neuroblastoma, melanoma, sarcoma, brain tumor, breast cancer or carcinoma.

88. The GD2 binding antibody of item 87, wherein said cancer is selected among osteosarcoma, liposarcoma, fibrosarcoma, malignant fibrous histiocytoma, leiomyosarcoma, spindle cell sarcoma, brain tumor, small cell lung cancer, retinoblastoma, HTLV-1 infected T cell leukemia, triple negative breast cancer and other GD2 positive tumors.

89. The GD2 binding antibody according to any of the items 80 - 88, wherein the GD2 binding antibody is a complete antibody consisting of two heavy chains and two light chains, a bispecific antibody, a SADA construct, an antibody fragment selected among F(ab')2, F(ab)2, Fab', Fab, Fv, scFv and the like, or a chimeric and/or recombinant construct comprising a GD2 binding site.

90. The GD2 binding antibody according to any of the items 80 - 89, wherein the GD2 binding antibody is an immunocytokine.

91. The GD2 binding antibody according to any of the items wherein 80 - 90, the GD2 binding antibody is humanized.

92. The GD2 binding antibody according to any of the items 80 - 91, wherein the GD2 binding antibody comprises the CDR sequences of SEQ ID NO: 5, 6, 7, 8, 9 and 10.

93. The GD2 binding antibody according to any of the items 80 - 92, comprising the VH sequence of SEQ ID NO: 3 and the VL sequence of SEQ ID NO: 4.

94. The GD2 binding antibody according to any of the items 80 - 93, comprising the heavy chain sequence of SEQ ID NO: 1 and the light chain sequence of SEQ ID NO: 2.

95. The GD2 binding antibody according to any of the items 80 - 94, comprising the sequence of SEQ ID NO: 11.

96. The GD2 binding antibody according to any of the items 80 - 95, wherein the administration in the first period is performed by infusion.

97. The GD2 binding antibody according to any of the items 80 - 96, wherein the first administration rate is selected so that dosage administered during step i. is 0.5-50%, 1-20%, preferably 2-15%, more preferred 3-12%, preferably 4-10%, more preferred 5-8%, preferably 6% w/w of the total dosage.

98. The GD2 binding antibody according to any of the items 80 - 97, wherein the first administration rate is below 250 $\mu$g/min.

99. The GD2 binding antibody according to any of the items 80 - 98, wherein the first administration rate is selected in the range of 50-250 $\mu$g/min, preferably 75-200 $\mu$g/min, preferably 100-150 $\mu$g/min preferably around 120 $\mu$g/min.

100. The GD2 binding antibody according to any of the items 80 - 99, wherein the first period is longer than 60 minutes, preferably in the range of 60-120 minutes, preferably in the range of 60 - 90 minutes and most preferred around 75 minutes.

101. The GD2 binding antibody according to any of the items 80 - 100, wherein the administration in the second period is performed by infusion.

102. The GD2 binding antibody according to any of the items 80 - 101, wherein the second administration rate is selected so that dosage administered during step ii. is at least 80% of the total administered dosage, preferably at least 90% or the total dosage, and most preferred around 94% of the total dosage.

103. The GD2 binding antibody according to any of the items 80 - 102, wherein the second administration rate is above 1 mg/min, preferably in the range of 1-25 mg/min, preferably in the range of 5-15 mg/min and most preferred around 10 mg/min.

104. The GD2 binding antibody according to any of the items 80 - 103, wherein the second period is 15 minutes or longer, preferably in the range of 15-30 minutes.

105. The GD2 binding antibody according to any of the items 80 - 104, wherein the total amount of GD2 binding antibody is administered in step i and ii.

106. The GD2 binding antibody according to item 105, wherein the total administered dosage of GD2 binding antibody is in the range of 50-150 mg.

107. The GD2 binding antibody according to any of the items 80 - 106, wherein the GD2 binding antibody is naxitamab, the first period is 75 minutes and the first administration rate is selected so 6% of the total administered dosage of GD2 binding antibody is administered in step i, and the rest of the total administered dosage is administered by infusion during step ii.

108. The GD2 binding antibody according to any of the items 80 - 107, further comprising a break between step i and step ii.

**Claims**

1. A GD2 binding antibody for use in a method of treatment or a diagnostic method comprising the administration to a subject of a dosage of said antibody, said method comprising the steps of:

   i. Administering said antibody with a first administration rate in a first period of time, followed by
   ii. Administering said antibody with a second administration rate in a second period of time, wherein said second administration rate is higher than said first administration rate;
   wherein the first administration rate is below 1 mg/kg/h;
   the first period is at least 5 minutes; and
   the total administration time of said dosage is not more than 6 hours, preferably not more than 4 hours.

2. The GD2 binding antibody of claim 1, wherein the first period is at least 10 minutes, preferably at least 15 minutes, more preferred at least 30 minutes, preferably at least 45 minutes, more preferred at least 45 minutes, preferably at least 60 minutes.

3. The GD2 binding antibody according to any of the preceding claims, comprising the CDR sequences of SEQ ID NO: 5, 6, 7, 8, 9 and 10.

4. The GD2 binding antibody according to any of the preceding claims, comprising a VH sequence and a VL sequence of SEQ ID NO: 3 and 4.

5. The GD2 binding antibody according to any of the preceding claims, wherein the diagnostic method or method of treatment is a method for diagnosing or treating cancer.

6. The GD2 binding antibody of claim 5, wherein the cancer is selected among neuroblastoma, melanoma, sarcoma, brain tumor, breast cancer or carcinoma.

7. The GD2 binding antibody of claim 6, wherein cancer is selected among osteosarcoma, liposarcoma, fibrosarcoma, malignant fibrous histiocytoma, leiomyosarcoma, spindle cell sarcoma, brain tumor, small cell lung cancer, retinoblastoma, HTLV-1 infected T cell leukemia, triple negative breast cancer and other GD2 positive tumors.

8. The GD2 binding antibody according to any of the preceding claims, wherein the dosage administered during step i. is 0.5-50%, 1-20%, preferably 2-15%, more preferred 3-12%, preferably 4-10%, more preferred 5-8%, preferably 6% w/w of the total dosage.

9. The GD2 binding antibody according to any of the preceding claims, wherein the first administration rate is selected in the range of 0.05-1 mg/kg/h, preferably 0.1-0.5 mg/kg/h, preferably around 0.3 mg/kg/h.

10. The GD2 binding antibody according to any of the preceding claims, wherein said second period of time is no more than 15 minutes, more preferred 30 minutes, preferably 45 minutes, more preferred 60 minutes, preferably 90 minutes.

11. The GD2 binding antibody according to any of the preceding claims, wherein the second administration rate is above 1 mg/kg/h, preferably in the range of 1-10 mg/kg/h, and most preferred around 4-6 mg/kg/h.

12. The GD2 binding antibody according to any of the preceding claims, wherein said dosage is not more than 3 mg/kg.

13. The GD2 binding antibody according to any of the preceding claims, wherein the antibody is administered with infusion, and the infusion rate is adjusted either A) within the following stages:

| Stage | Infusion Time (minutes) | Rate (mg/kg/h) |
|---|---|---|
| 1 | 15 | 0.06 |

(continued)

| Stage | Infusion Time (minutes) | Rate (mg/kg/h) |
|---|---|---|
| 2 | 15 | 0.12 |
| 3 | 15 | 0.24 |
| 4 | 15 | 0.48 |
| 5 | 15 | 0.96 |
| 6 | 15 | 1.92 |
| 7 | 32 | 3.84 |

Or B) within the following stages:

| Stage | Infusion Time (minutes) | Rate (mg/kg/h) |
|---|---|---|
| 1 | 15 | 0.24 |
| 2 | 15 | 0.48 |
| 3 | 15 | 0.96 |
| 4 | 15 | 1.92 |
| 5 | 15 | 3.84 |
| 6 | 11 | 6.00 |

**14.** The GD2 binding antibody according to any of the preceding claims, wherein the antibody is administered with infusion, and the dose is administered either A) in the following stages:

| Stage | Infusion Time (minutes) | Dose administered in stage (mg/kg) |
|---|---|---|
| 1 | 15 | 0.015 |
| 2 | 15 | 0.03 |
| 3 | 15 | 0.06 |
| 4 | 15 | 0.12 |
| 5 | 15 | 0.24 |
| 6 | 15 | 0.48 |
| 7 | 32 | 2.05 |

Or B) in the following stages:

| Stage | Infusion Time (minutes) | Dose administered in stage (mg/kg) |
|---|---|---|
| 1 | 15 | 0.06 |
| 2 | 15 | 0.12 |
| 3 | 15 | 0.24 |
| 4 | 15 | 0.48 |
| 5 | 15 | 0.96 |
| 6 | 11 | 1.14 |

**15.** The GD2 binding antibody according to any of the preceding claims for use in a method of treatment or a diagnostic method, wherein the subject of said administration is a human adult, a juvenile or a child.

Fig. 1

Fig. 2

Fig. 3

Fig. 4

Fig. 5

Dose Administered in Each Stage Day 3 and 5

Fig. 6

Fig. 7

Cumulative Dose for Each of Day 3 and 5

Fig. 8

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/134801 A1 (LOIBNER HANS [AT] ET AL) 17 May 2018 (2018-05-17)<br>* e.g. page 2, left-hand column, paragraph 2; claim 1;<br>the whole document * | 1-15 | INV.<br>C07K16/30<br>A61P35/00<br>A61K39/00 |
| X | US 2015/139942 A1 (LOIBNER HANS [AT] ET AL) 21 May 2015 (2015-05-21)<br>* e.g. paragraph 12; claim 1;<br>the whole document * | 1-15 | |
| X | DORALINA L. ANGHELESCU ET AL: "Comparison of pain outcomes between two anti-GD2 antibodies in patients with neuroblastoma",<br>PEDIATRIC BLOOD AND CANCER,<br>8 November 2014 (2014-11-08), pages n/a-n/a, XP055250272,<br>US<br>ISSN: 1545-5009, DOI: 10.1002/pbc.25280<br>* e.g. abstract;<br>the whole document * | 1-15 | |
| X | CA 2 834 000 A1 (APEIRON BIOLOGICS AG [AT]) 21 May 2015 (2015-05-21)<br>* e.g. paragraph bridging pages 3 and 4; claim 1;<br>the whole document * | 1-15 | TECHNICAL FIELDS SEARCHED (IPC)<br><br>C07K<br>A61K |

The present search report has been drawn up for all claims

| Place of search | Date of completion of the search | Examiner |
|---|---|---|
| The Hague | 2 May 2022 | Gruber, Andreas |

EPO FORM 1503 03.82 (P04C01)

## ANNEX TO THE EUROPEAN SEARCH REPORT
## ON EUROPEAN PATENT APPLICATION NO.

EP 21 38 3131

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2022

| Patent document cited in search report | | Publication date | Patent family member(s) | | Publication date |
|---|---|---|---|---|---|
| US 2018134801 | A1 | 17-05-2018 | AU | 2012383254 A1 | 20-11-2014 |
| | | | BR | 112014031806 A2 | 29-08-2017 |
| | | | CA | 2876529 A1 | 27-12-2013 |
| | | | CN | 104487088 A | 01-04-2015 |
| | | | EA | 201500019 A1 | 29-05-2015 |
| | | | HK | 1203375 A1 | 30-10-2015 |
| | | | JP | 6283665 B2 | 21-02-2018 |
| | | | JP | 2015521607 A | 30-07-2015 |
| | | | KR | 20150030650 A | 20-03-2015 |
| | | | SG | 10201610532Q A | 27-02-2017 |
| | | | SG | 11201408487W A | 27-02-2015 |
| | | | US | 2014170155 A1 | 19-06-2014 |
| | | | US | 2018134801 A1 | 17-05-2018 |
| | | | US | 2020055953 A1 | 20-02-2020 |
| | | | WO | 2013189516 A1 | 27-12-2013 |
| | | | WO | 2013189554 A1 | 27-12-2013 |
| | | | ZA | 201407961 B | 28-10-2015 |
| US 2015139942 | A1 | 21-05-2015 | EP | 2861251 A1 | 22-04-2015 |
| | | | US | 2015139942 A1 | 21-05-2015 |
| CA 2834000 | A1 | 21-05-2015 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018204873 A1

**Non-patent literature cited in the description**

- Pathogenesis of the neurotoxicity caused by anti-GD2 antibody therapy. *Journal of the Neurological Sciences,* 01 August 1997, vol. 149, 127-130
- **YUKI et al.** reported side effects associated with administration of anti-GD2. *MAb (14G2a)*
- **BLOM, THOMAS et al.** Treatment-Related Toxicities During Anti-GD2 Immunotherapy in High-Risk Neuroblastoma Patients. *Frontiers in oncology,* 17 February 2021, vol. 10, 601076
- **KUSHNER.** Humanized 3F8 Anti-GD2 Monoclonal Antibody Dosing with Granulocyte-Macrophage Colony-Stimulating Factor in Patients with Resistant Neuroblastoma A Phase 1 Clinical Trial. *JAMA Oncology,* 20 September 2018, vol. 4, 1729-1735
- **FURMAN.** A Phase II Trial of Hu14.18K322A in Combination with Induction Chemotherapy in Children with Newly Diagnosed High-Risk Neuroblastoma. *Clinical Cancer Research,* 25 November 2019

- **ALBERTINI.** A Pilot Trial of Hu14.18-IL2 (EMD 273063) in Subjects with Completely Resectable Recurrent Stage III or Stage IV Melanoma. *Cancer Immunology Immunotherapy,* October 2018, vol. 67, 1647-1658
- **MURRAY.** Phase I trial of murine monoclonal antibody 14G2a administered by prolonged intravenous infusion in patients with neuroectodermal tumors. *Journal of Clinical Oncology,* 01 January 1994, vol. 12, 184-193
- **MURRAY et al.** *Mab 14G2a has modest antitumor activity at the expense of significant toxicity.*
- **NAVID, FARIBA et al.** Anti-GD2 antibody therapy for GD2-expressing tumors. *Current cancer drug targets,* 2010, vol. 10 (2), 200-9
- Phase I Trial of a Novel Anti-GD22 Monoclonal Antibody, Hu14.18K322A, Designed to Decrease Toxicity in Children With Refractory or Recurrent Neuroblastoma. *J. Clin. Oncol.,* 2014, vol. 32, 1445-1452
- European Pharmacopoeia